# EUROPEAN PATENT SPECIFICATION

(11) **EP 3 815 777 B1**
(45) Date of publication and mention of the grant of the patent: **02.08.2023**
(21) Application number: 19206082.0
(22) Date of filing: 30.10.2019
(51) Int. Cl.: B01F 31/40, B01F 33/501, B01F 35/71, B01F 35/75, A61B 17/88, B67B 7/92, B01F 101/20

(54) **MIXING AND DISPENSING SYSTEM AND METHOD OF MIXING AND DISPENSING MATERIAL FROM A MIXING AND DISPENSING SYSTEM**
MISCH- UND AUSGABESYSTEM UND VERFAHREN ZUM MISCHEN UND AUSGEBEN VON MATERIAL AUS EINEM MISCH- UND AUSGABESYSTEM
SYSTÈME DE MÉLANGE ET DE DISTRIBUTION ET PROCÉDÉ DE MÉLANGE ET DE DISTRIBUTION DE MATÉRIAU À PARTIR D'UN SYSTÈME DE MÉLANGE ET DE DISTRIBUTION

(43) Date of publication of application: 05.05.2021
(73) Proprietor: medmix Switzerland AG, 9469 Haag (Rheintal) (CH)
(72) Inventor: NIEBER, Benjamin, 6274 Eschenbach (CH); MOSER, Joseph, 5444 Künten (CH); MATHYS, Beat, 5630 Muri (CH); VEID, Martin, 6353 Weggis (CH)
(74) Representative: Manitz Finsterwald Patent- und Rechtsanwaltspartnerschaft mbB

(56) References cited:
- WO-A1-2014/202350
- DE-B3-102014 108 569
- JP-A- 2008 113 903
- KR-B1- 101 275 638
- US-A1- 2016 016 134

## Description

The present invention relates to a mixing and dispensing system for a two-component material, such as a bone cement, the mixing and dispensing system comprising a handle connected to the mixer via a mixing shaft, wherein the mixing shaft extends through the piston from the piston driving end in the direction of the outlet end in order to move the mixer in dependence on movements initiated via the handle, and with the handle being detachable, in particular in a non-destructive manner, from the mixing shaft via a connector. The invention further relates to a method of mixing and dispensing material from a mixing and dispensing system. Mixing and dispensing systems are known to mix two-component materials, such as the ones used in joint replacement surgery. On mixing two-component material, such as cement, e.g. PMMA (poly-methyl methacrylate) used e.g. in joint replacement surgery, such as hip replacement surgery, cracks in the cement mantle may facilitate a mechanical loosening of the prosthesis.

Moreover, the cement is to be used in a sterile environment and may be rather harmful to the user of the mixing and dispensing system which is why the possible contact of the user with the cement prepared in the mixing and dispensing system is not desirable.

For this reason it is an object of the invention to provide a mixing and dispensing system in which the two-component material can be sufficiently mixed, such that no fatigue cracks occur in the solidified two-component material and in which the contact a user of the mixing and dispensing system may have with the two-component material to be mixed can be minimized.

Prior art devices are known from JP2008113903A, KR101275638B1, WO2014/202350A1, US2016/016134A1, and DE102014108569B3, which discloses a mixing and dispensing system according to the preamble of claim 1.

This object is satisfied by a mixing and dispensing system having the features of claim 1.

Such a mixing and dispensing system for a two-component material, such as a bone cement, comprises a cartridge having a piston driving end and an outlet end and a chamber formed therebetween for storage of at least a first component of the two-component material, a piston arranged moveable to and fro between the piston driving end and the outlet end and stored initially at the piston driving end, a mixer arranged within the chamber between the piston and the outlet end, wherein the mixer is moveable relative to the piston and the chamber and a handle connected to the mixer via a mixing shaft, wherein the mixing shaft extends through the piston from the piston driving end in the direction of the outlet end in order to move the mixer in dependence on movements initiated via the handle, and with the handle being detachable, in particular in a non-destructive manner, from the mixing shaft via a connector, the handle further comprising a rod, the rod being journaled by said mixing shaft and the rod being detachable from the mixing shaft via the connector.

The mixer is provided with the cartridge in order to mix the two-component material within the cartridge to achieve the desired mixing results and to avoid the provision of further components that would require increased cost of manufacture and would further be required to be produced in a sterile environment.

The handle can initiate both rotational and translational movements of the mixer in order to thoroughly mix the two-component materials. Following the mixing the handle can then be removed in a non-destructive and/or tool-free manner via the connector.

In this way a pre-loaded and sterile cartridge of the mixing and dispensing system can be made available which is particularly useful in a medical environment of use. Moreover, since the handle can be removed by way of a connector, there is no danger of a user of the mixing and dispensing system coming into contact with the mixed two-component material.

The handle comprises a rod and the rod is detachable from the mixing shaft via said connector. Such rods can easily be inserted into mixing shafts and may have an outer shape complementary to an inner shape of the mixing shaft which at least in regions is non-symmetrical in order to transmit both rotational and translational forces from the rod to the mixing shaft.

The connector may be part of a drive interface for applying translational as well as rotational forces at the mixer via said rod and mixing shaft, such as a drive interface formed by a tight fit respectively a force fit. In this way both translational as well as rotational forces can be transmitted from the handle to the mixer.

The rod may comprise elements more flexible than the mixing shaft at the connector. Such elements can be used to disconnect the rod from the mixing shaft in a simple manner.

The connector may comprise one or more buttons that can be depressed to disengage the handle from the mixing shaft. The provision of such buttons makes available well defined points of activation of the connector for detaching the rod respectively the handle from the mixing shaft.

The one or more buttons may be connected to the elements more flexible than the mixing shaft. In this way the elements which have to be actuated to disconnect the rod from the mixing shaft are in direct connection with the buttons which initiate the disconnection of the rod from the mixing shaft.

The connector, i.e. the buttons thereof, may be arranged outside of the cartridge. In this way the access to the connector can be achieved in a simple manner.

The rod may extend from the handle beyond the connector towards the mixer within said mixing shaft. In this way a stable connection between the rod and the mixing shaft in the region of the connector can be achieved.

By way of example between 10 and 50%, in particular 15 to 40% of a length of the rod may project beyond the connector into the mixing shaft in order to facilitate a stable connection between the rod and the mixing shaft.

The handle may be arranged at least substantially perpendicular to the mixing shaft. In this way a user can beneficially grip the handle relative to the mixing shaft in order to initiate both rotational and translation movements of the mixer.

The mixing shaft may be journaled by said piston. This means that the mixing shaft may pass through the piston between the handle and the mixer. This facilitates a smaller construction of the cartridge which produces less waste material on producing and using such cartridges.

The rod is journaled by said mixing shaft. This means that the rod is received within the mixing shaft rather than the mixing shaft being received within the rod. The mixer may be moveable axially along a longitudinal axis between the piston and the outlet end and is rotatable about the longitudinal axis. In this way beneficial mixing results of the two-component material to be mixed can be achieved as rotational as well as translational movements of the mixer can be carried out in the cartridge.

The piston may be releasably locked in a storage position at the piston driving end prior to mixing with said mixer; and the piston may be moveable via a dispensing mechanism, such as a threaded dispenser, following the release of the piston from the storage position for dispensing said two-component material from said cartridge. In this way a construction space of the cartridge can be further reduced reducing the waste material following use of the mixing and dispensing system, since the piston is stored in the cartridge. Moreover, through the use of a dispensing mechanism, the two-component material can be dispensed in an expedient and accurate manner.

The mixer may comprise two or more vanes extending radially outward from the mixing shaft. Such vanes enable an improved mixing of the two-component material in the cartridge.

The mixer may comprise two or more vanes extending radially outward from the mixing shaft, said two or more vanes may be connected to an outer ring. The provision of an outer ring can stabilize the mixer and hence lead to improved mixing results in comparison to mixers without such outer rings.

The outlet end may comprise a first interface via which an ampoule container and a dispensing outlet can be releasably connected to the cartridge. The piston driving end may comprise a second interface via which a dispensing mechanism can be connected to the cartridge after the handle has been removed from the mixing shaft. The provision of such first and second interfaces ensures a reliable and quick exchange of the respective components at the mixing and dispensing system, which particularly in a surgical environment where the medical professionals use gloves etc. can be particularly beneficial.

According to a further aspect the present invention relates to a method of mixing and dispensing material from a mixing and dispensing system according to claim 1, the method comprising the steps of:
- moving the mixer in dependence on movements initiated via the handle, and detaching the handle from the mixing shaft via the connector.

By way of such a method the handle can be reliably removed from the mixing and dispensing system while the user does not come into contact with the mixed two-component material, such that pre-loaded and sterile mixed two-component materials can be made available in a sterile cartridge which is particularly useful in a medical environment of use.

Further embodiments of the invention are described in the following description of the Figures. The invention will be explained in the following in detail by means of embodiments and with reference to the drawings in which is shown:
- Fig. 1a: a side view of components of a mixing and dispensing system;
- Fig. 1b: a sectional view taken along the sectional line B:B of Fig. 1a of the mixing and dispensing system;
- Fig. 2a: an enlarged view of the activation element of Fig. 1a;
- Fig. 2b: an enlarged view of the section through the activation element of Fig. 1b;
- Fig. 3a: an enlarged sectional view of a handle of the mixing and dispensing system shown in Fig. 1b;
- Fig. 3b: an enlarged side view of the handle of Fig. 3a;
- Fig. 3c: an exploded view of the handle of Fig. 3b partly removed from a mixing shaft;
- Fig. 4a: a sectional view similar to the one of Fig. 1b with a threaded dispenser and a dispensing outlet arranged at a cartridge of a mixing and dispensing system, said system not being a system according to the invention;
- Fig. 4b: a part sectional part schematic view of the threaded dispenser of Fig. 4a;
- Fig. 4c: an enlarged view of the section shown in Fig. 4a in the region of a piston driving end of the cartridge;
- Fig. 4d: a part sectional part schematic view of first and second threads of the threaded dispenser of Fig. 4a;
- Fig. 4e: an enlarged partial view of part of the threaded dispenser of Fig. 4a;
- Fig. 4f: a sectional view taken along the section A:A of Fig. 4e; and
- Fig. 4g: an enlarged view of part of the section of Fig. 4f.

In the following the same reference numerals will be used for parts having the same or equivalent function. Any statements made having regard to the direction of a component are made relative to the position shown in the drawing and can naturally vary in the actual position of application.

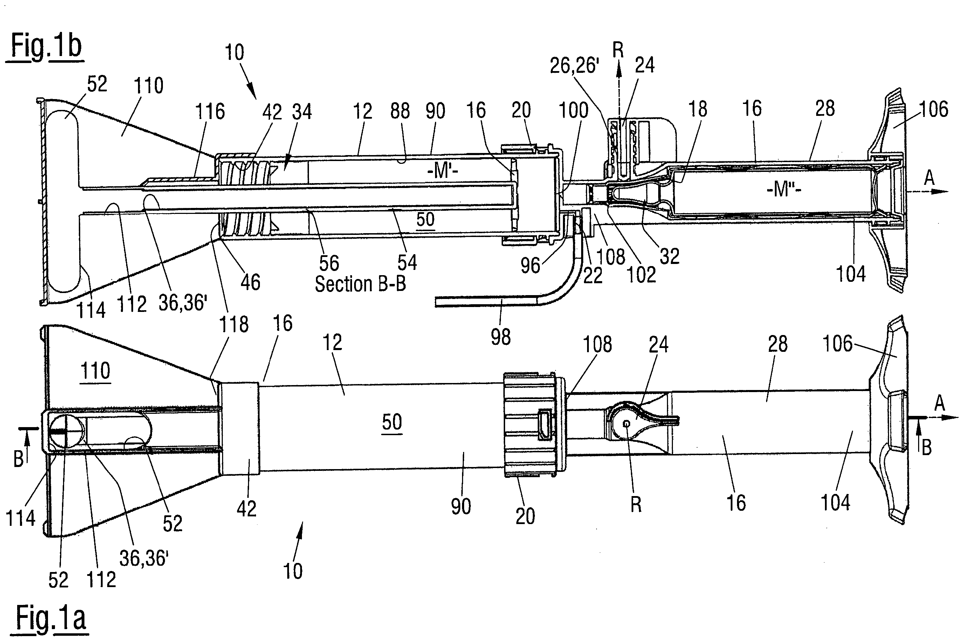

Fig. 1a shows a side view of components of a mixing and dispensing system 10 for mixing a two-component material M', M", such as a bone cement. Fig. 1b shows a sectional view taken along the sectional line B:B of Fig. 1a of the mixing and dispensing system 10.

The mixing and dispensing system 10 comprises a cartridge 12 for storage of a first component M' of the two-component material M', M", the cartridge 12 comprising a mixer 14 arranged therein.

An ampoule container 16 for receiving an ampoule 18 comprising a second component M" of the two-component material M', M" is connected to the cartridge 12 via a first interface 20. The first interface 20 is configured to releasably connect and disconnect the cartridge 12 with and from said ampoule container 16. A longitudinal axis A extends between said cartridge 12 and said ampoule container 16 and through said first interface 20.

The ampoule container 16 further comprises an activation element 24. On moving said activation element 24 from a storage position into an activated position, a part 26 of said activation element 24 moves perpendicular to said longitudinal axis A such that the part 26 contacts a top 32 of said ampoule 18 for opening said ampoule 18 when said activation element 24 is displaced from the storage position into the activated position. Thereby the second component M" stored in said ampoule 18 can be released from said ampoule 18 into said cartridge 12 to come into contact with said first component M' stored in said cartridge 12 for mixing the two-component material M', M" in said cartridge 12 with said mixer 14.

The activation element 24 is arranged partly at an outer wall 28 of the ampoule container 16. More specifically a rotatable switch 30, such as a half turn coupling 30', of the activation element 24 is arranged at the outer wall 28. The activation element 24 further comprises an activation plunger 26' arranged within said ampoule container 16. The activation plunger 26' is configured to contact the top 32 of said ampoule 18 for opening said ampoule 18 when said activation element 24 is displaced from the storage position into the activated position.

In this connection also a quarter turn coupling or the like would be feasible to activate the activation plunger 26'.

In order to displace the activation plunger 26' from the storage position into the activated position, the rotatable switch 30 is rotated about an axis of rotation R to produce an axial movement of said activation plunger 26' along said axis of rotation R of said rotatable switch 30.

A vacuum port 22 is also arranged at the ampoule container 16 for providing a vacuum within the mixing and dispensing system 10. The vacuum port 22 is arranged remote from the activation element 24 in such a way that it does not come into contact with the rotatable switch 30 on rotating the rotatable switch 30 about the axis of rotation R.

On mixing two-component material M', M", such as cement M, M", e.g. PMMA (poly-methyl methacrylate) used e.g. in joint replacement surgery, such as hip replacement surgery, cracks in the cement mantle may facilitate a mechanical loosening of the prosthesis. In order to avoid this a vacuum is applied on mixing the cement M', M" in the cartridge 12 to reduce the cement porosity of the cement such that less cracks, pores etc. are formed in the cement and particularly its mantle.

In this connection it should be noted that the cartridge 12 should be sealed off to the outside during the mixing of the two-component material M', M", such that a contamination or the like of the two-component material M', M" can be avoided.

For this purpose the vacuum port 22 is arranged at the outer wall 28 of the ampoule container 16 such that it can evacuate a chamber 50 of the cartridge 12 formed between a piston driving end 46 and an outlet end 48 of the cartridge 12.

In order to evacuate the chamber 50, a groove 96, in particular an L-shaped groove 96, leads from the vacuum port 22 to the chamber 50 through the first interface 20 to the cartridge 12. The vacuum port 22 can be connected to a source of vacuum (not shown) via a vacuum line 98 in a manner known per se.

In this connection it should be noted that a corresponding passage or groove can also be provided extending from the vacuum port 22 to the chamber 50 if the vacuum port 22 is arranged at the cartridge 12 rather than at the ampoule container 16.

It should further be noted in this connection that it is preferred to have the vacuum port 22 at the ampoule container 16 since this makes the handling of the cartridge 12 simpler after mixing and disconnecting of the ampoule container 16, since no vacuum port 22 would then be present at the cartridge 12.

The mixer 14 is also arranged within the chamber 50 between the piston 34 and the outlet end 48, with the mixer 14 being moveable relative to the piston 34 and the chamber 50 during mixing of the cement M', M". The mixer 14 can be moved radially about said longitudinal axis A and axially along said longitudinal axis A for mixing said two-component material M', M".

A piston 34 is arranged moveable to and fro between the piston driving end 46 and the outlet end 48 of the cartridge and is stored initially at the piston driving end 46. The piston 34 is pre-positioned within the cartridge 12 such that once a force above a threshold pressure is applied on the piston 34, the piston 34 can dispense the mixed two-component material M', M" from the cartridge. Such forces can typically be selected in the range of 100 to 3000 N.

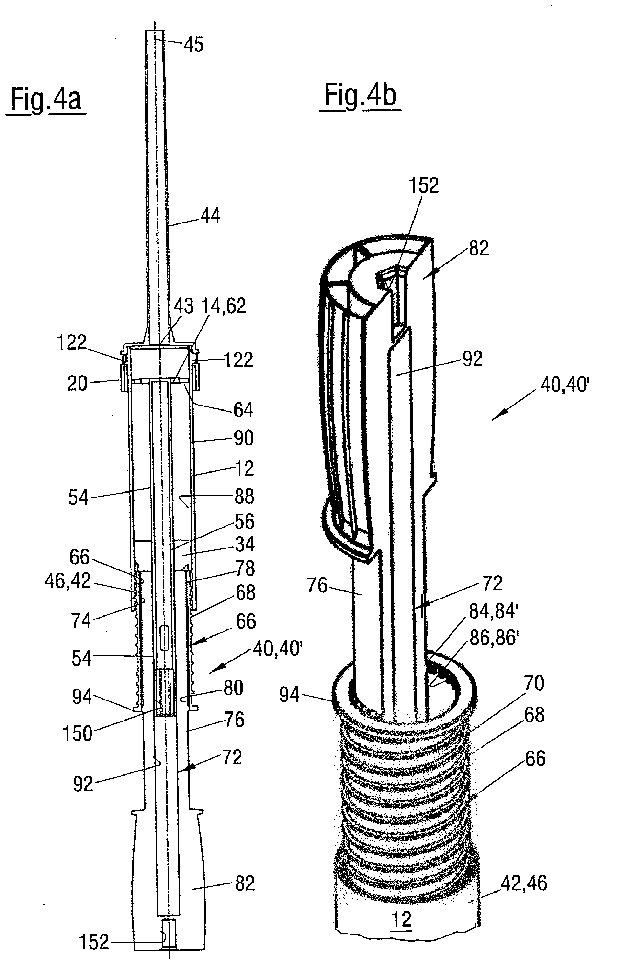

The piston 34 may be moveable via a threaded dispenser 40 (see Fig. 4a) following the release of the piston 34 from the storage position for dispensing said two-component material M', M" from said cartridge 12.

Once the ampoule 18 has been opened the second component M" stored therein is moved into the cartridge 12 via a passage 100 arranged at the ampoule container 16 for mixing in the chamber 50.

In order to avoid glass splinters (not shown) from being moved together with the second component M" into the cartridge 12, a net 102 is arranged around the top 32 of the ampoule 18.

A cap 106 can be placed onto the end 104 of the ampoule container 16 remote from the end 108 present at the first interface 20. The cap 106 can act as a stand for the ampoule container 16, such that on storing and/or activating the mixing and dispensing system 10, the mixing and dispensing system 10 can stand on a surface via this cap 106.

A part 36, more specifically a rod 36', of the mixer 14 is detachable via a connector 38 of a drive interface 38', in such a way that the threaded dispenser 40 of a dispensing mechanism 40' (see Fig. 4a) can be connected to the mixing and dispensing system 10 via a second interface 42 of the cartridge 12 at the piston driving end 46.

The mixer 14 is connected to a handle 52 via a mixing shaft 54 and the rod 36'. The mixing shaft 54 extends between the handle 52 and the mixer 14 and passes through the piston 34 via a piston passage 56 such that the mixer 14 can be moved in dependence on movements initiated via the handle 52 and indeed relative to the piston 34 and the chamber 50.

The handle 52 is detachable, in particular in a non-destructive manner, from the mixing shaft 54 via the connector 38. In this connection it should be noted that the handle 52 can also be removed without the use of tools, i.e. in a tool-free manner.

On storing the mixing and dispensing system 10, the handle 52 and part of the mixing shaft 54 are stored in a stand 110 that comprises respective grooves 112, 114 for receiving and storing the handle 52 and part of the mixing shaft 54. An end 118 of the stand is arranged adjacent to the piston driving end 46 in the storage state of the mixing and dispensing system 10. As indicated in Fig. 1b, the stand 110 may comprise a support 116 to reinforce the stand 110 and to ensure the handle 52 and thus the mixer 14 is not moved in the storage state of the mixing and dispensing system 10.

In this connection it should be noted that the precise position of the mixer 14 within the cartridge 12 is not pre-defined in the storage state of the mixing and dispensing system 10, the stand 110 should merely ensure that the mixer 14 does not move in the storage state of the mixing and dispensing system 10.

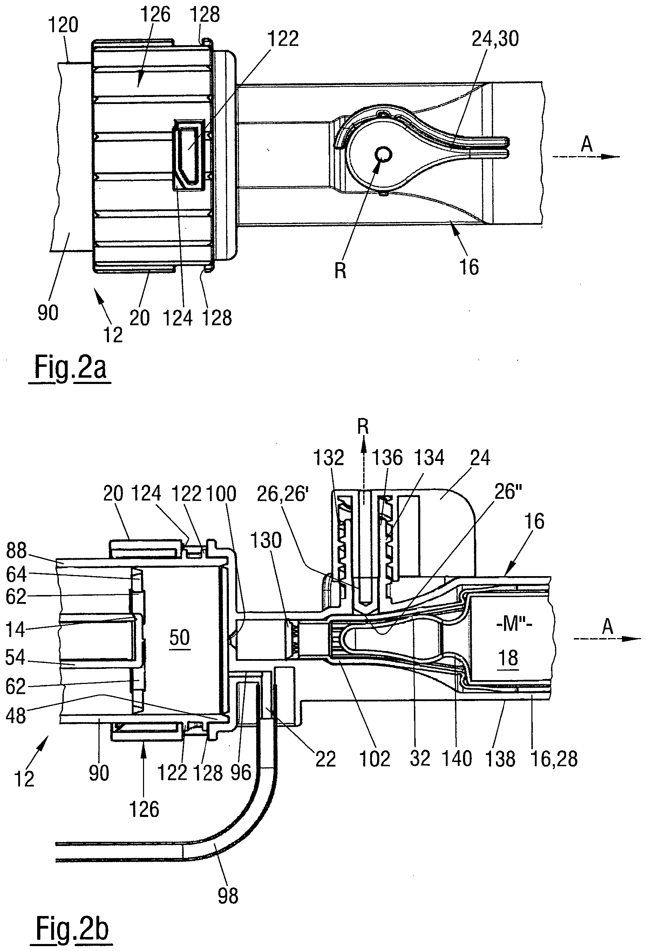

Fig. 2a shows an enlarged view of the first interface 20 and of the activation element 24 of Fig. 1a. The first interface 20 is formed by a bayonet type interface in order to releasably couple the ampoule container 16 to the cartridge 12. As indicated in Fig. 4a also a dispensing outlet 44 can be releasably coupled to the cartridge 12 via the first interface 20.

To form the first interface 20 an outer surface 120 of the cartridge 12 comprises pins 122 extending radially therefrom. The pins 122 engage matching slots 124 (see also Fig. 2b) present in a cap 126 of the first interface 20. In Fig. 2a apertures 128 of the slots 124 are visible. In this connection it should be noted that between 2 and 6, preferably between 2 and 4 pins 122 with matching slots 124 can be provided. The cap 126 in this way sits on the outlet end 48 of the cartridge 12.

A distance between the cap 126 and the activation element 24 shown is such that the switch 30 can be rotated by at most approximately 270° relative to the ampoule container 16. This means that a distance the activation plunger 26' can travel between the storage position and the activation position has to be achieved by at most a 270° degree turn of the rotatable switch 30, with the illustrated design of the rotatable switch 30.

If more turns are desired then a distance between the cap 126 and the rotatable switch 30 has to be increased, such that these components do not interfere with one another.

Additionally or alternatively, the activation element 24 could be snap fit into place and the rotation thereof can be used solely for displacing the activation plunger 26' along the axis of rotation R.

In the present instance, it has been found to be prudent to use a half turn coupling as the rotatable switch 30 as in this way one achieves a maximum distance the plunger 26' can travel in a well-defined manner through a minimum rotation of the rotatable switch 30 to achieve the opening of an ampoule 18 stored in the ampoule container 16.

Fig. 2b shows an enlarged view of the section through the first interface 20 and the activation element 24 of Fig. 1b. A membrane 130 is visible between the top 32 of the ampoule and the cap 126. This membrane 130 aids in preventing glass splinters from arriving in the cartridge 12.

As is further evident in the sectional drawing indicated in Fig. 2b, the plunger 26' is arranged at the rotatable switch 30. To bring about the rotation of the rotatable switch 30, this comprises an inner thread 132, which can rotate on an outer thread 134 of a hollow projection 136 projecting radially outwardly from an outer surface 138 of the ampoule container 16.

The plunger 26' projects from within the rotatable switch 30 through the outer wall 28 of the ampoule container 16 via the hollow projection 136, so that a tip 26" of the plunger 26' can contact the top 32 of the ampoule 18 above a neck 140 of the ampoule 18 in order to break and/or open the ampoule 18.

For the purpose of breaking and/or opening the top 32 of the ampoule, the tip 26" may have a specific shape, such as the frustoconical shape shown in Fig. 2b, or also have a conical shape or have a rounded shape.

In this connection it should be noted that the rotatable switch 30 may be integrally formed together with the plunger 26' having the tip 26" as one piece in an injection molding process.

The specific design of the mixer 14 is also shown in Fig. 2b. The mixer 14 is moveable axially along the longitudinal axis A between the piston 34 and the outlet end 48 and is rotatable about the longitudinal axis A. In order to carry out the mixing the mixer comprises several vanes 62 extending radially outward from the mixing shaft 54. At their radial outer ends the vanes 62 are connected to an outer ring 64. By way of this mixer 14 design the two-component material M', M" can be thoroughly mixed. Also other designs of mixers 14 are possible (not shown).

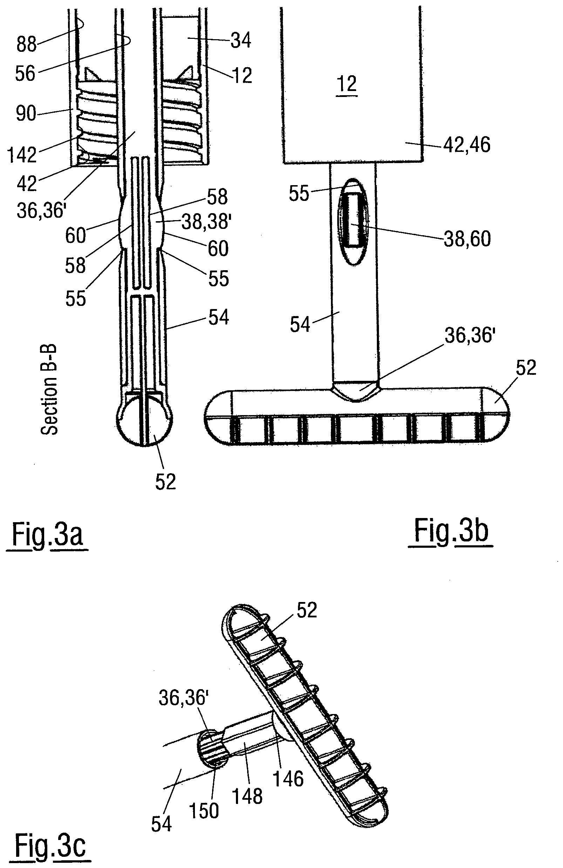

Fig. 3a shows an enlarged sectional view of the handle 52 of the mixing and dispensing system 10 shown in Fig. 1b. The mixing shaft 54 extends between the handle 52 and the mixer 14 through the piston 34 via the piston passage 56 and into the cartridge 12. The mixing shaft 54 is journaled by said piston 34 in the piston passage 56.

To connect the handle 52 to the mixing shaft 54, a rod 36' that is non-releasably connected to the handle 52 projects into said mixing shaft 54 and is thereby journaled by said mixing shaft 54. The rod 36' extends from the handle 52 beyond the connector 38 towards the mixer 14 within said mixing shaft 54.

In order to release the handle 52 from the mixing shaft 54, the connector 38 has to be arranged outside of the cartridge 12 for actuation thereof. On releasing the handle 52 two buttons 60 have to be depressed to disengage the handle 52 from the mixing shaft 54.

This is possible in the present example, since the rod 36' comprises elements 58 more flexible than the mixing shaft 54 at the connector 38. On pressing the buttons 60, the buttons 66 press the elements 58 such that they deflect inwardly so that the rod 26' of the handle can disengage from the mixing shaft 54.

This is made possible, since the elements 58 are able to deflect relative to the mixing shaft 54 and hence are more flexible than the mixing shaft 54, due to less material being provided and the elements 58 therefore being less rigid than the mixing shaft 54.

Also other methods of releasably and non-destructively releasing the handle 52 from the mixing shaft are possible, for example using threaded components (not shown).

Part of the second interface 42 is also indicated in Fig. 3a, where an inner thread 142 is present at an inner surface 80 of a cartridge wall 90 of the cartridge 12, via which second interface 42 the threaded dispenser 40 (see Fig. 4a) can be releasably coupled to the cartridge 12.

Fig. 3b shows an enlarged side view of the handle of Fig. 3a. The handle 52 is formed by a bar 144 in this example, but also other shapes are possible, such as a knob or the like. The bar 144 of the handle 52 is arranged perpendicular to the mixing shaft 54.

Fig. 3c shows an exploded view of the handle 52 of Fig. 3b partly removed from a mixing shaft 54. The connector 38 is part of a drive interface 38' for applying translational as well as rotational forces initiated via said handle 52 at the mixer 14 specifically via said rod 36' and mixing shaft 54.

For this purpose and as indicated in Fig. 3c an end 146 of the rod 36 connected to the handle 52 may comprise a hexagonal fitting 148. The hexagonal fitting 148 engages a hexagonal shaped socket 150 formed in the mixing shaft 54. In this way both translational and rotational forces can be transmitted from the handle 52 to the mixer 14 via this tight fit respectively force fit detachable connection. In this connection it should be noted that also other kinds of detachable connections are possible provided they permit a transmission of rotational and translational forces from the handle 52 to the mixer 14.

Fig. 4a shows a sectional view similar to the one of Fig. 1b with the threaded dispenser 40, i.e. a dispensing mechanism 40', arranged at the piston driving end 46 and the dispensing outlet 44 arranged at the outlet end 48 of the cartridge 12 of the mixing and dispensing system 10.

The dispensing outlet 44 is releasably connectable to said cartridge 12 via said first interface 20 on removal of said ampoule container 16 (see Figs. 1a to 2b). The dispensing outlet 44 shown has an inlet 43 at its end adjacent the first interface 20 and an outlet 45 at its opposite end, with an inner diameter of the dispensing outlet 44 tapering from the inlet 43 towards the outlet 45, such that the outlet 45 has a smaller diameter than the inlet 43. The dispensing outlet 44 thus has a frustoconical shape. In this connection it should be noted that also other shapes of dispensing outlets 44 can be provided (not shown).

The dispensing mechanism 40' comprises a sleeve 66 having a first thread 68 arranged at an outer surface 70 thereof for connecting the sleeve 66 to the piston driving end 46 of the cartridge 12. The dispensing mechanism 40' further comprises a cartridge plunger 72 that can be arranged partly within said sleeve 66, the cartridge plunger 72 being moveable with and relative to said sleeve 66, and the cartridge plunger 72 having a second thread 74 arranged at an outer surface 76 thereof. The second thread 74 can interact with an inner thread 78 present at an inner surface 80 of the sleeve 66. The cartridge plunger 72 comprises an engagement part 82 for rotating at least the cartridge plunger 72 and depending on the state of use of the threaded dispenser 40' also the sleeve 66.

Fig. 4b shows a part sectional part schematic view of the threaded dispenser 40 of Fig. 4a. First and second holding members 84, 86 are present between the cartridge plunger 72 and the sleeve 66. The first and second holding members 84, 86 are configured to hold the cartridge plunger 72 at the sleeve 66 when a torque below a pre-defined threshold is applied at the cartridge plunger 72 in a manner similar to a torque wrench. Moreover, the first and second holding members 84, 86 are configured to release the cartridge plunger 72 from the sleeve 66 once a torque above a pre-defined threshold is applied at the cartridge plunger 72.

In this connection it should be noted that the pre-defined threshold can be selected in the range of 0.2 to 1.2 Nm.

The first holding member 84 is formed at an outer surface 76 of the cartridge plunger 72 and comprises a plurality of teeth 84'. The second holding member 86 is formed at the inner surface 80 of the sleeve 66 and comprises a plurality of cams 86'. The plurality of teeth 84' and the plurality of cams 86' are formed complementary to one another.

The plurality of teeth 84' are configured to slip over the plurality of cams 86' when a torque applied at said cartridge plunger 72 exceeds the pre-defined threshold, in a manner similar to a slipper type torque wrench.

The engagement part 82 is formed as a handle in the embodiment shown. As the case may be the engagement part 82 may comprise a coupling point 152 such as a hexagonal socket for connecting the dispensing mechanism 40', i.e. the threaded dispenser 40 to a further component, such as a power tool or a hand tool for rotating the cartridge plunger 72.

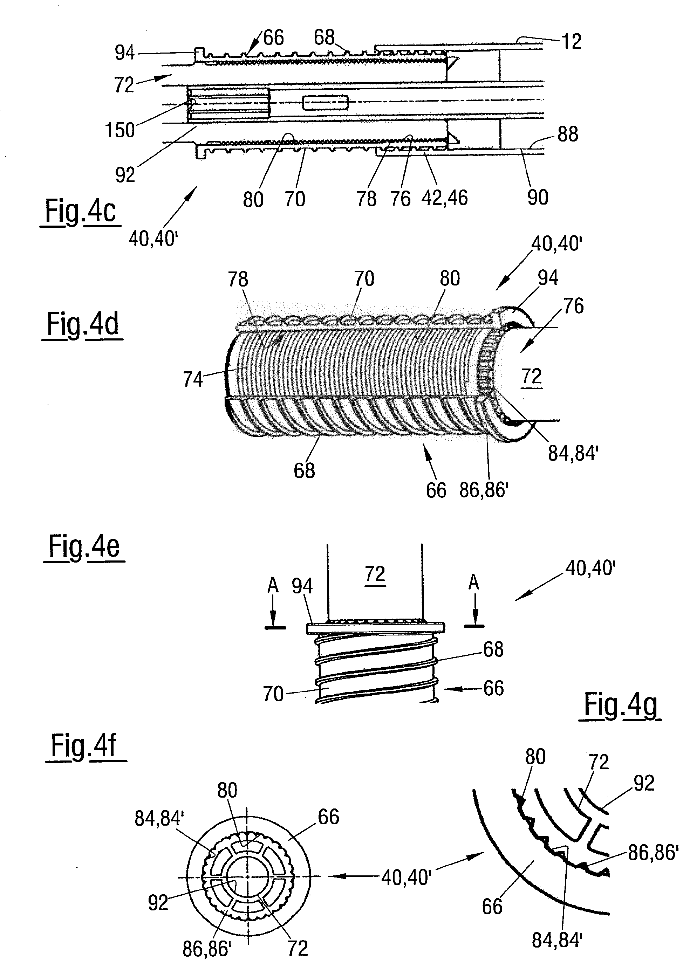

Fig. 4c shows an enlarged view of the section shown in Fig. 4a in the region of the piston driving end 46 of the cartridge 12. The first thread 68 is configured to engage the inner surface 88 of the cartridge wall 90 of the cartridge 12.

On moving the sleeve 66 into the cartridge 12 it is configured to release the piston 34 present in said cartridge 12 from a storage position of said piston 34. Once the sleeve 66 has arrived at its final position in the cartridge 12, the cartridge plunger 72 experiences a resistance so that the torque required to turn the cartridge plunger 72 further in the direction of the longitudinal axis A exceeds the pre-defined threshold, whereby the cartridge plunger 72 disengages from the sleeve 66 at the first and second holding members 84, 86 and slips out of engagement from the sleeve 66.

Then the second thread 74 starts to turn relative to the sleeve 66 in the inner thread 78 present at the inner surface 80 of the sleeve 66. This moves the cartridge plunger 72 further along the longitudinal axis A into the cartridge 12. The cartridge plunger 72 is configured to move the piston 34 present in said cartridge 12 to and fro along the longitudinal axis A of said cartridge 12 once the sleeve 66 has released the piston 34 from the storage position of said piston.

On releasing the handle 52 from the mixing shaft 54, neither the mixer 14 nor the mixing shaft 54 are removed, the threaded dispenser 40 is moved over the mixing shaft 54, which is received in a passage 92 configured for receiving the mixing shaft 54.

Fig. 4d shows a part sectional part schematic view of the first and second threads 68, 74 of the threaded dispenser 40 of Fig. 4a. The first and second threads 68, 74 have different pitches, with the second thread 74 being finer than the first thread 68, i.e. the second thread 74 has a lower thread pitch than the first thread 68.

Fig. 4e shows an enlarged partial view of part of the threaded dispenser 40 of Fig. 4a. The sleeve 66 has an abutment 94 at an end thereof remote to the end that is initially introduced into the cartridge 12. The abutment 94 comes into contact with the piston driving end 46 of the cartridge 12 once the sleeve 66 has arrived at its final position in the cartridge 12. This is typically the position in which the torque on the cartridge plunger 72 can reach the pre-defined threshold in order to disengage the cartridge plunger 72 from the sleeve 66.

Fig. 4f shows a sectional view taken along the section A:A of Fig. 4e. In this drawing like in Fig. 4e one can see that the abutment 94 is arranged at the same axial height as the second holding member 86 which in turn may be formed by the plurality of cams 86' within the sleeve 66.

Fig. 4g shows an enlarged view of part of the section of Fig. 4f. In this view both the plurality of cams 86' as well as the plurality of teeth 84' respectively forming the second and first holding members 86, 84 are visible. If a torque less than a pre-defined threshold is applied at the plunger then the teeth 84' mesh with the cams 86' to rotate the sleeve 66. If a torque exceeding the pre-defined threshold is applied, then the teeth 84' slip over the cams 86' so that the cartridge plunger 72 can move relative to the sleeve 66.

On activating the mixing and dispensing system 10 illustrated in Figs. 1a and 1b, the activation element 24 is moved from the storage position into the activated position, thereby the part 26, i.e. the activation plunger 26', of said activation element 24 is moved perpendicular to said longitudinal axis A and comes into contact with the top 32 of the ampoule 18 to open this and to release the second component M" stored therein. The second component M" is brought into contact with the first component M' in said cartridge 12. The first and second components M', M" of the two-component material M', M" are then mixed in the chamber 50 of the cartridge 12 with said mixer 14.

To mix the two-component material M', M", the mixer is moved via the mixing shaft 54 and handle 52 to and fro along the longitudinal axis A and by rotating the handle 52, the mixer is also rotated about the longitudinal axis A. The vanes 62 and the outer ring 64 bring about a thorough through mixing of the two-component material M', M", as the mixer is moved through the chamber 50 and through the two-component material M', M".

It should be noted that the handle 52 and/or the mixing shaft 54 may have a socket, such as the hexagonal socket 150, via which the mixer 14 and the mixing shaft 54 can be set into rotation using e.g. a power tool. In this way more uniform mixing results of the two-component material M', M" can possibly be achieved.

In this connection it should be noted that the vacuum applied via the vacuum port 22 can be applied either before or during opening of the ampoule 18. If the vacuum is applied before breaking of the ampoule 18, this has the advantage that movement of the second component M" stored in the ampoule 18 into the cartridge 12 can be assisted through the use of the vacuum as soon as the ampoule 18 is open as the vacuum so to say sucks the second component M" into the cartridge 12.

Moreover, due to the presence of the vacuum the amount of air and thus pores in the mixed two-component material M', M" is significantly reduced, so that less air is present in the mixture reducing the amount of fatigue cracks that may appear in the solidified two-component material M', M", leading to improved adhesion results between the two-component material M', M" and the surfaces (not shown) between which it is applied.

Following the mixing of the two-component material M', M" with the mixing and dispensing system 10 the handle 52 connected to the mixer 14 via the mixing shaft 54 is detached. For this purpose a user presses the buttons 60 present at the connector 38. By pressing the buttons 60 the elements 58 more flexible than the mixing shaft 54 present at the rod 36' allow the rod 36' to disengage from the mixing shaft 54 in the regions of the apertures 55 present in the mixing shaft 54, whereby the rod 36' can be removed from the mixing shaft 54 to detach the handle 52 from the mixing shaft 54 via the connector 38.

In order to dispense material from the mixing and dispensing system 10 the user has to remove the ampoule container 16 from the cartridge 12 via the first interface 20. Following which the user can arrange a dispensing outlet 44, such as the dispensing outlet 44 shown in Fig. 4a, at the cartridge 12 using the first interface 20.

Following this the user now has several options to press the mixed two-component material M', M" out of the chamber 50 of the cartridge 12. One option is to install an insert (not shown) at the second interface 42, via which a dispensing gun can be connected to the cartridge 12, for dispensing the two-component material M', M" from the cartridge 12.

The other option is to use the dispensing mechanism 40' discussed in relation to Figs. 4a to 4g. This is done by attaching the sleeve 66 at the second interface 42, by turning the cartridge plunger 72, such that the first thread 68 of the sleeve 66 is turned into the inner thread 142. During the connection of the sleeve 66 to the cartridge the force on the sleeve 66 is below a pre-defined threshold value such that the sleeve 66 is entrained by the first and second holding members 84, 86 and transmits the rotations of the cartridge plunger 72 onto the sleeve 66. Once the abutment 94 contacts the cartridge 12, the sleeve 66 is finally installed at the second interface 42. As the sleeve 66 reaches its final position it also releases the piston 34 from the storage position for dispensing said two-component material M', M" from said cartridge 12.

In this final position the sleeve 66 experiences a resistance at the cartridge which means that an increased force has to be applied at the cartridge plunger 72 in order to rotate this further. This means that a force above the pre-defined threshold value is applied at the cartridge plunger 72, such that the first holding members 84 slip past the second holding members 86 so that the second thread 74 present at the outer surface outer surface 76 of the plunger can interact with the inner thread 78 present at an inner surface 80 of the sleeve 66.

The cartridge plunger 72 then comes into contact with the piston 34 for dispensing said two-component material M', M" from said cartridge 12. Once the cartridge plunger 72 has slipped past the first and second holding members 84, 86 one rotates only the cartridge plunger 72 via the engagement part 82. The engagement part 82 can be rotated either by hand or using a power tool (not shown) connectable to the engagement part via the coupling point 152.

On moving the piston 34 via the cartridge plunger 72, the mixer 14 may at least at some point of the dispensing process rest on the piston 34 and be entrained via the piston 34.

The dispensing mechanism 40' is designed in a way that allows a fast forwarding at the beginning, i.e. while the sleeve 66 is attached to the cartridge 12 via the second interface 42, and once the torque above a pre-defined threshold is achieved changes over to the cartridge plunger 72 having a lower pitch which transmits higher forces when needed for dispensing the two-component material M', M" via the dispensing outlet 44.

In this connection it should be noted that a material of at least one of the cartridge 12, the ampoule container 16, the cap 126 of the first interface 20, the cap 106 and the stand 110 and of the piston 34 may be polypropylene (PP).

A material of at least one of the handle 52, the rotatable switch 30, the rod 36', and the threaded dispenser 40 may be one of PP and polyamide (PA).

The net 102 and of the membrane 130 may be formed of a thermoplastic elastomer (TPE).

Using the above mixing and dispensing system 10 a user, such as a medical professional, can minimize exposure to the two-component material M', M". If, for example PMMA is used as a Monomer then this Monomer can be disturbing for the respiratory tract. The use of the ampoule container described in the foregoing, the disconnectable handle and the dispensing mechanism 40' reduces the risk of the medical professional coming into contact with the monomer. Moreover, if the dispensing mechanism 40' is used one can use the mixing and dispensing system 10 independent of a multiple-use dispensing gun.

### List of reference numerals:

- 10: mixing and dispensing system
- 12: cartridge
- 14: mixer
- 16: ampoule container
- 18: ampoule
- 20: first interface
- 22: vacuum port
- 24: activation element
- 26, 26': part of 24, activation plunger
- 28: outer wall of 16
- 30, 30': rotatable switch, quarter turn coupling
- 32: top of 18
- 34: piston
- 36, 36': part of 14, rod
- 38, 38': connector, drive interface
- 40, 40': threaded dispenser
- 42: second interface
- 43: inlet of 44
- 44: dispensing outlet
- 45: outlet of 44
- 46: piston driving end
- 48: outlet end
- 50: chamber
- 52: handle
- 54: mixing shaft
- 55: aperture
- 56: piston passage
- 58: elements
- 60: button
- 62: vane
- 64: outer ring
- 66: sleeve
- 68: first thread
- 70: outer surface of 66
- 72: cartridge plunger
- 74: second thread
- 76: outer surface of 72
- 78: inner thread of 66
- 80: inner surface of 66
- 82: engagement part
- 84, 84': first holding member, tooth
- 86, 86': second holding member, cam
- 88: inner surface of 12
- 90: cartridge wall
- 92: passage
- 94: abutment
- 96: groove
- 98: vacuum line
- 100: passage
- 102: net
- 104: end
- 106: cap
- 108: end
- 110: stand
- 112: groove for 54
- 114: groove for 52
- 116: support of 110
- 118: end of 110
- 120: outer surface of 12
- 122: pin
- 124: slot
- 126: cap
- 128: aperture
- 130: membrane
- 132: inner thread of 30
- 134: outer thread
- 136: projection
- 138: outer surface of 16
- 140: neck of 18
- 142: inner thread of 12
- 144: bar
- 146: end
- 148: hexagonal fitting
- 150: hexagonal socket
- 152: coupling point

- A: longitudinal axis
- M', M": first component of material, second component of material
- R: axis of rotation of 30

## Claims

1. A mixing and dispensing system (10) for a two-component material (M', M"), such as a bone cement, the mixing and dispensing system (10) comprising:
- a cartridge (12) having a piston driving end (46) and an outlet end (48) and a chamber (50) formed therebetween for storage of at least a first component (M') of the two-component material (M', M"),
- a piston (34) arranged moveable to and fro between the piston driving end (46) and the outlet end (48) and stored initially at the piston driving end (46);
- a mixer (14) arranged within the chamber (50) between the piston (34) and the outlet end (48), wherein the mixer (14) is moveable relative to the piston (34) and the chamber (50); and
- a handle (52) connected to the mixer (14) via a mixing shaft (54), wherein the mixing shaft (54) extends through the piston (34) from the piston driving end (46) in the direction of the outlet end (48) in order to move the mixer (14) in dependence on movements initiated via the handle (52), and with the handle (52) comprising a rod (36'), wherein the rod is journaled by said mixing shaft (54),
**characterized in that**
the rod (36') is detachable from the mixing shaft (54) via a connector (38).

2. A mixing and dispensing system (10) according to claim 1,
wherein the connector (38) is part of a drive interface (38') for applying translational as well as rotational forces at the mixer (14) via said rod (36') and mixing shaft (54), such as a drive interface formed by a tight fit respectively a force fit.

3. A mixing and dispensing system (10) according to claim 1 or claim 2,
wherein the rod (36') comprises elements (58) more flexible than the mixing shaft (54) at the connector (38).

4. A mixing and dispensing system (10) according to one of the preceding claims, wherein the connector (38) comprises one or more buttons (60) that can be depressed to disengage the handle (52) from the mixing shaft (54).

5. A mixing and dispensing system (10) according to claim, wherein the rod (36') comprises elements (58) more flexible than the mixing shaft (54) at the connector (38) and wherein the one or more buttons (60) are connected to the elements (58) more flexible than the mixing shaft (54).

6. A mixing and dispensing system (10) according to one of the preceding claims,
wherein the connector (38) is arranged outside of the cartridge (12).

7. A mixing and dispensing system (10) according to one of claims 1 to 6, wherein the rod (36') extends from the handle (52) beyond the connector (38) towards the mixer (14) within said mixing shaft (54).

8. A mixing and dispensing system (10) according to one of the preceding claims,
wherein the handle (52) is arranged at least substantially perpendicular to the mixing shaft (54).

9. A mixing and dispensing system (10) according to one of the preceding claims,
wherein the mixing shaft (54) is journaled by said piston (34).

10. A mixing and dispensing system (10) according to one of the preceding claims,
wherein the mixer (14) is moveable axially along a longitudinal axis (A) between the piston (34) and the outlet end (48) and is rotatable about the longitudinal axis (A).

11. A mixing and dispensing system (10) according to one of the preceding claims,
wherein the piston (34) is releasably locked in a storage position at the piston driving end (46) prior to mixing with said mixer (14); and the piston (34) is moveable via a dispensing mechanism (40'), such as a threaded dispenser (40), following the release of the piston (34) from the storage position for dispensing said two-component material (M', M") from said cartridge (12).

12. A mixing and dispensing system (10) according to one of the preceding claims, wherein the mixer (14) comprises two or more vanes (62) extending radially outward from the mixing shaft (54).

13. A mixing and dispensing system (10) according to one of the preceding claims, wherein the mixer (14) comprises two or more vanes (62) extending radially outward from the mixing shaft (54), said two or more vanes being connected to an outer ring (64).

14. A mixing and dispensing system (10) according to one of the preceding claims, wherein the outlet end (48) comprises a first interface (20) via which an ampoule container (16) and a dispensing outlet (44) can be releasably connected to the cartridge (12).

15. A mixing and dispensing system (10) according to one of the preceding claims, wherein the piston driving end (46) comprises a second interface (42) via which a dispensing mechanism (40') can be connected to the cartridge (12) after the handle (52) has been removed from the mixing shaft (54).

16. A method of mixing and dispensing material (M', M") from a mixing and dispensing system (10) in accordance with one of the preceding claims, the method comprising the steps of:
- moving the mixer (14) in dependence on movements initiated via the handle (52), and detaching the handle (52) from the mixing shaft (54) via the connector (38).

## Patentansprüche

1. Misch- und Abgabesystem (10) für ein Zweikomponentenmaterial (M', M"), wie einen Knochenzement, wobei das Misch- und Abgabesystem (10) umfasst:
- eine Kartusche (12) mit einem Kolbenantriebsende (46) und einem Auslassende (48) und einer dazwischen gebildeten Kammer (50) zum Speichern von mindestens einer ersten Komponente (M') des Zweikomponentenmaterials (M', M"),
- einen Kolben (34), der zwischen dem Kolbenantriebsende (46) und dem Auslassende (48) hin- und herbewegbar angeordnet ist und zunächst am Kolbenantriebsende (46) gelagert ist;
- einen Mischer (14), der innerhalb der Kammer (50) zwischen dem Kolben (34) und dem Auslassende (48) angeordnet ist, wobei der Mischer (14) relativ zu dem Kolben (34) und der Kammer (50) beweglich ist); und
- einen Griff (52), der über eine Mischwelle (54) mit dem Mischer (14) verbunden ist,
wobei sich die Mischwelle (54) durch den Kolben (34) vom Kolbenantriebsende (46) in Richtung des Auslassendes (48) erstreckt, um den Mischer (14) in Abhängigkeit von Bewegungen zu bewegen, die über den Griff (52) eingeleitet werden, und wobei der Griff (52) eine Stange (36') umfasst, wobei die Stange durch die Mischwelle (54) gelagert ist,
**dadurch gekennzeichnet, dass**
die Stange (36') über ein Verbindungsstück (38) von der Mischwelle (54) abnehmbar ist.

2. Misch- und Abgabesystem (10) nach Anspruch 1,
wobei das Verbindungsstück (38) Teil einer Antriebsschnittstelle (38') ist, um sowohl translatorische als auch rotatorische Kräfte über die Stange (36') und die Mischwelle (54) auf den Mischer (14) aufzubringen, wie eine Antriebsschnittstelle, die durch einen Festsitz bzw. einen Kraftsitz gebildet ist.

3. Misch- und Abgabesystem (10) nach einem der Ansprüche 1 oder 2, wobei die Stange (36') Elemente (58) aufweist, die flexibler sind als die Mischwelle (54) am Verbindungsstück (38).

4. Misch- und Abgabesystem (10) nach einem der vorhergehenden Ansprüche, wobei das Verbindungsstück (38) einen oder mehrere Knöpfe (60) umfasst, die gedrückt werden können, um den Griff (52) von der Mischwelle (54) zu lösen.

5. Misch- und Abgabesystem (10) nach Anspruch, wobei die Stange (36') Elemente (58) aufweist, die flexibler sind als die Mischwelle (54) am Verbindungsstück (38) und wobei der eine oder die mehreren Knöpfe (60) mit den Elementen (58) verbunden sind, die flexibler sind als die Mischwelle (54).

6. Misch- und Abgabesystem (10) nach einem der vorhergehenden Ansprüche,
wobei das Verbindungsstück (38) außerhalb der Kartusche (12) angeordnet ist.

7. Misch- und Abgabesystem (10) nach einem der Ansprüche 1 bis 6,
wobei sich die Stange (36') vom Griff (52) über das Verbindungsstück (38) hinaus in Richtung des Mischers (14) innerhalb der Mischwelle (54) erstreckt.

8. Misch- und Abgabesystem (10) nach einem der vorhergehenden Ansprüche,
wobei der Griff (52) zumindest im Wesentlichen senkrecht zur Mischwelle (54) angeordnet ist.

9. Misch- und Abgabesystem (10) nach einem der vorhergehenden Ansprüche,
wobei die Mischwelle (54) durch den Kolben (34) gelagert ist.

10. Misch- und Abgabesystem (10) nach einem der vorhergehenden Ansprüche,
wobei der Mischer (14) axial entlang einer Längsachse (A) zwischen dem Kolben (34) und dem Auslassende (48) beweglich ist und um die Längsachse (A) drehbar ist.

11. Misch- und Abgabesystem (10) nach einem der vorhergehenden Ansprüche,
wobei der Kolben (34) vor dem Mischen mit dem Mischer (14) in einer Lagerposition am Kolbenantriebsende (46) lösbar verriegelt ist; und der Kolben (34) über einen Abgabemechanismus (40'), wie eine mit einem Gewinde versehene Abgabeeinrichtung (40), nach dem Lösen des Kolbens (34) aus der Lagerposition beweglich ist, um das Zweikomponentenmaterial (M', M") aus der Kartusche (12) auszugeben.

12. Misch- und Abgabesystem (10) nach einem der vorhergehenden Ansprüche, wobei der Mischer (14) zwei oder mehr Schaufeln (62) aufweist, die sich von der Mischwelle (54) radial nach außen erstrecken.

13. Misch- und Abgabesystem (10) nach einem der vorhergehenden Ansprüche, wobei der Mischer (14) zwei oder mehr Schaufeln (62) aufweist, die sich von der Mischwelle (54) radial nach außen erstrecken, wobei die zwei oder mehr Schaufeln mit einem Außenring (64) verbunden sind).

14. Misch- und Abgabesystem (10) nach einem der vorhergehenden Ansprüche, wobei das Auslassende (48) eine erste Schnittstelle (20) aufweist, über die ein Ampullenbehälter (16) und ein Abgabeauslass (44) lösbar mit der Kartusche (12) verbunden werden können.

15. Misch- und Abgabesystem (10) nach einem der vorhergehenden Ansprüche, wobei das Kolbenantriebsende (46) eine zweite Schnittstelle (42) aufweist, über die ein Abgabemechanismus (40') mit der Kartusche (12) verbunden werden kann, nachdem der Griff (52) von der Mischwelle (54) entfernt wurde.

16. Verfahren zum Mischen und Ausgeben von Material (M', M") aus einem Misch- und Abgabesystem (10) nach einem der vorhergehenden Ansprüche, wobei das Verfahren die Schritte umfasst:
- Bewegen des Mischers (14) in Abhängigkeit von Bewegungen, die über den Griff (52) eingeleitet werden, und
- Lösen des Griffs (52) von der Mischwelle (54) über das Verbindungsstück (38).

## Revendications

1. Système de mélange et de distribution (10) pour un matériau à deux composants (M', M"), tel qu'un ciment osseux, le système de mélange et de distribution (10) comprenant :
- une cartouche (12) ayant une extrémité d'entraînement de piston (46) et une extrémité de sortie (48) et une chambre (50) formée entre celles-ci pour stocker au moins un premier composant (M') du matériau à deux composants (M', M"),
- un piston (34) agencé de manière mobile en va-et-vient entre l'extrémité d'entraînement de piston (46) et l'extrémité de sortie (48) et stocké initialement au niveau de l'extrémité d'entraînement de piston (46) ;
- un mélangeur (14) agencé à l'intérieur de la chambre (50) entre le piston (34) et l'extrémité de sortie (48), le mélangeur (14) pouvant être déplacé relativement au piston (34) et à la chambre (50) ; et
- une poignée (52) connectée au mélangeur (14) via un arbre de mélange (50),
dans lequel l'arbre de mélange (54) s'étend à travers le piston (34) depuis l'extrémité d'entraînement de piston (46) dans la direction de l'extrémité de sortie (48) afin de de déplacer le mélangeur (14) en dépendance de mouvements initiés via la poignée (52), et la poignée (52) comprenant une tige (36'), la tige étant tourillonnée par ledit arbre de mélange (54),
**caractérisé en ce que**
la tige (36') peut être détachée de l'arbre de mélange (54) via un connecteur (38).

2. Système de mélange et de distribution (10) selon la revendication 1,
dans lequel le connecteur (38) est une partie d'une interface d'entraînement (38') pour appliquer des forces de translation aussi bien que de rotation au niveau du mélangeur (14) via ladite tige (36') et ledit arbre de mélange (54), telle qu'une interface d'entraînement formée par un ajustement de serrage, respectivement un ajustement de force.

3. Système de mélange et de distribution (10) selon la revendication 1 ou 2, dans lequel la tige (36') comprend des éléments (58) plus flexibles que l'arbre de mélange (54) au niveau du connecteur (38).

4. Système de mélange et de distribution (10) selon l'une des revendications précédentes, dans lequel le connecteur (38) comprend un ou plusieurs boutons (60) qui peuvent être enfoncés pour désengager la poignée (52) de l'arbre de mélange (54).

5. Système de mélange et de distribution (10) selon la revendication, dans lequel la tige (36') comprend des éléments (58) plus flexibles que l'arbre de mélange (54) au niveau du connecteur (38) et dans lequel lesdits un ou plusieurs boutons (60) sont connectés aux éléments (58) plus flexibles que l'arbre de mélange (54).

6. Système de mélange et de distribution (10) selon l'une des revendications précédentes, dans lequel le connecteur (38) est agencé à l'extérieur de la cartouche (12).

7. Système de mélange et de distribution (10) selon l'une des revendications précédentes 1 à 6, dans lequel la tige (36') s'étend depuis la poignée (52) au-delà du connecteur (38) vers le mélangeur (14) à l'intérieur dudit arbre de mélange (54).

8. Système de mélange et de distribution (10) selon l'une des revendications précédentes, dans lequel la poignée (52) est agencée au moins sensiblement perpendiculairement à l'arbre de mélange (54).

9. Système de mélange et de distribution (10) selon l'une des revendications précédentes, dans lequel l'arbre de mélange (54) est tourillonné par ledit piston (34).

10. Système de mélange et de distribution (10) selon l'une des revendications précédentes, dans lequel le mélangeur (14) peut être déplacé axialement le long d'un axe longitudinal (A) entre le piston (34) et l'extrémité de sortie (48) et peut être mis en rotation autour de l'axe longitudinal (A).

11. Système de mélange et de distribution (10) selon l'une des revendications précédentes, dans lequel le piston (34) peut être verrouillé de manière libérable dans une position de stockage au niveau de l'extrémité d'entraînement de piston (46) avant le mélange avec ledit mélangeur (14) ; et le piston (34) peut être déplacé via un mécanisme de distribution (40'), tel qu'un distributeur à pas de vis (40), à la suite de la libération du piston (34) de la position de stockage pour distribuer ledit matériau à deux composants (M, M') depuis ladite cartouche (12).

12. Système de mélange et de distribution (10) selon l'une des revendications précédentes, dans lequel le mélangeur (14) comprend deux ou plusieurs palettes (62) s'étendant radialement vers l'extérieur depuis l'arbre de mélange (54).

13. Système de mélange et de distribution (10) selon l'une des revendications précédentes, dans lequel le mélangeur (14) comprend deux ou plusieurs palettes (62) s'étendant radialement vers l'extérieur depuis l'arbre de mélange (54), lesdites deux ou plusieurs palettes (62) étant connectées à une bague extérieure (64).

14. Système de mélange et de distribution (10) selon l'une des revendications précédentes, dans lequel l'extrémité de sortie (48) comprend une première interface (20) via laquelle un récipient à ampoule (16) et une sortie de distribution (44) peuvent être connectés à la cartouche (12) de manière libérable.

15. Système de mélange et de distribution (10) selon l'une des revendications précédentes, dans lequel l'extrémité d'entraînement de piston (46) comprend une seconde interface (42) via laquelle un mécanisme de distribution (40') peut être connecté à la cartouche (12) après que la poignée (52) a été retirée de l'arbre de mélange (54).

16. Procédé de mélange et de distribution de matériau (M, M') provenant d'un système de mélange et distribution (10) selon l'une quelconque des revendications précédentes, le procédé comprenant les étapes consistant à :
- déplacer le mélangeur (14) en dépendance de mouvements initiés par la poignée (52), et
- détacher la poignée (52) de l'arbre de mélange (54) via le connecteur (38).
